# DEMANDE DE BREVET EUROPEEN

(11) **EP 3 192 543 A1**
(43) Date de publication de la demande: **19.07.2017**
(21) Numéro de dépôt: 16151684.4
(22) Date de dépôt: 18.01.2016
(51) Int. Cl.: A61M 1/16, A45C 15/00, B62B 1/14

(54) **DISPOSITIF COMPRENANT UNE BOÎTE ET AU MOINS UN OBJET LOGÉ DANS CETTE BOÎTE**

(71) Demandeur: Infomed SA, 1227 Geneve (CH)
(72) Inventeur: Negrevergne, Carole, 74140 Machilly (FR)
(74) Mandataire: Micheli & Cie SA

(57) **Abrégé**

La présente invention a pour objet un dispositif (1) comprenant une boîte (2) et au moins un objet (4) destiné à être logé dans ladite boîte (2), ladite boîte (2) comprenant une ouverture (5) pour laisser passer ledit au moins un objet (4) et un système de levage (3). Ledit système de levage (3) est conçu pour recevoir ledit au moins un objet (4) de manière fixe et permet i) de sortir ledit au moins un objet (4) de la boîte (2) en le faisant passer à travers ladite ouverture (5), typiquement pour l'utiliser, et ii) de rentrer ledit au moins un objet (4) entièrement dans la boîte (2), typiquement pour le ranger ou pour le transporter.

## Description

### DOMAINE DE L'INVENTION

La présente invention concerne un dispositif comprenant une boîte et au moins un objet, par exemple un appareil médical, logé dans cette boîte.

### ETAT DE LA TECHNIQUE

Certains objets tels que les appareils de soin à domicile ou les appareils médicaux sont lourds et fragiles et doivent pourtant voyager régulièrement.

De tels objets nécessitent un transport sans risques de casse, doivent arriver prêts à l'utilisation et pouvoir être installés par les patients eux-mêmes. Il est donc indiqué qu'ils soient protégés durant le transport et que leur poids, qui est souvent supérieur à 10 kilos, ne pose pas un problème de manipulation pour les patients qui sont par définition affaiblis.

En général, ces appareils sont transportés jusqu'aux patients emballés dans des boîtes, par exemple dans des boîtes en matériau renforcé ou moulées aux formes de l'appareil à transporter. Lorsque l'emballage arrive chez le patient, ce dernier doit le déballer, fournir un effort physique ou utiliser un outil de levage (lequel n'est pas forcément disponible sur les lieux) pour extraire l'appareil de son emballage et l'installer à bonne hauteur pour pouvoir l'utiliser.

Toutes ces opérations requièrent une énergie dont les patients ne sont pas toujours capables, impliquent de trouver un meuble ou tout autre support pour placer l'appareil à la bonne hauteur et impliquent également que l'appareil ne bouge plus une fois en place, restant en permanence en vue du patient et de ses éventuels proches.

Les patients conservent généralement les boîtes de ces appareils pour pouvoir les retourner après utilisation, boîtes qui ne servent pourtant à rien durant l'utilisation de l'appareil et deviennent donc inutiles et encombrantes. Ces emballages ne sont parfois même pas réutilisables et finissent comme déchets encombrants.

De plus, lorsqu'ils souhaitent réintroduire l'appareil dans sa boîte les patients ont les mêmes difficultés que celles rencontrées pour son extraction.

Les difficultés pour le transport d'appareils médicaux ou d'appareils de soins à domicile, tels que les appareils de dialyse, précédemment décrites se rencontrent également pour le transport de nombreux autres objets, typiquement pour le transport de tout objet relativement fragile, lourd et/ou volumineux, et dont l'utilisation est, de préférence, ponctuelle. On peut citer par exemple le cas d'appareils de mesure en extérieur.

Un objectif de l'invention est donc de fournir un dispositif qui permette de protéger efficacement l'objet lors de son transport et de faciliter sa réception, sa mise en service (mise en place, utilisation effective, rangement), et son transport à petite ou grande échelle.

Un objectif accessoire de l'invention est de fournir un dispositif encore plus particulièrement adapté au transport d'appareils médicaux tels que, par exemple, les appareils de dialyse.

D'autres avantages seront mis en avant dans la description ci-dessous.

### RESUME DE L'INVENTION

A ces fins, l'invention propose un dispositif comprenant une boîte et au moins un objet destiné à être logé dans ladite boîte, ladite boîte comprenant une ouverture pour laisser passer ledit au moins un objet et un système de levage, ledit système de levage étant conçu pour recevoir ledit au moins un objet de manière fixe et permettant i) de sortir ledit au moins un objet de la boîte en le faisant passer à travers ladite ouverture, typiquement pour l'utiliser, et ii) de rentrer ledit au moins un objet entièrement dans la boîte, typiquement pour le ranger ou pour le transporter.

De préférence, la boîte du dispositif comprend en outre un couvercle agencé pour fermer l'ouverture de la boîte.

Dans un mode de réalisation préféré, le système de levage du dispositif selon l'invention permet de régler la hauteur de l'objet hors de la boîte. Un tel mode de réalisation permet un réglage de la hauteur de l'objet adaptée aux besoins de l'utilisateur. Par exemple, dans le cas d'un dispositif comprenant une boîte équipée d'un système de levage sur lequel est fixé un appareil de dialyse, le patient peut régler la hauteur de l'appareil de dialyse selon qu'il opère l'appareil allongé, assis ou debout. Pour ce faire, il n'est donc pas obligé de trouver un meuble ou tout autre support possédant une hauteur convenable pour placer son appareil de dialyse.

Dans un mode de réalisation particulier, la boîte du dispositif comporte au moins un moyen de commande permettant de piloter le système de levage.

De préférence, la boîte du dispositif selon l'invention est, au moins en partie, moulée aux formes dudit au moins un objet.

Avantageusement, la boîte est fabriquée dans une matière synthétique propre à absorber les chocs et les vibrations dues au transport, par exemple en polypropylène expansé(PPE).

Dans un mode de réalisation préféré de l'invention, la boîte du dispositif comporte des roulettes. De préférence, lesdites roulettes ne dépassent pas de la surface de la boîte lorsque celle-ci repose sur l'une quelconque de ses faces mais permettent de faire rouler ladite boîte lorsque celle-ci est inclinée, par exemple entre 14 et 80 degrés. De préférence, la boîte du dispositif selon l'invention comporte également une poignée télescopique. Ces éléments permettent au dispositif selon l'invention d'être déplaçable sans effort, notamment sur des courtes distances, facilitant ainsi sa livraison et son utilisation, par exemple à l'intérieur d'un logement.

De manière préférée, la boîte du dispositif selon l'invention comprend également au moins une poignée fixe permettant de soulever le dispositif, par exemple, pour le mettre dans un véhicule.

Dans un mode particulier de réalisation de l'invention, le système de levage comporte un moteur électrique, lequel peut être alimenté par une batterie ou sur secteur.

De manière préférée, ledit au moins un objet du dispositif selon l'invention est un appareil médical, par exemple un appareil de dialyse. Avantageusement, la boîte du dispositif comprend un bac de rétention porté par le système de levage et situé sous ledit appareil médical. En effet, certains appareils, et en particulier ceux manipulant des compositions liquides, comme par exemple des fluides corporels, sont associés à un risque de fuite. La présence d'un bac de rétention sous de tels appareils présente l'avantage de protéger le dispositif ainsi que le sol de ces éventuelles fuites de liquide. Dans le cas particulier de la dialyse à domicile, l'appareil nécessite entre 15 et 40 litres de soluté fournis en poches. Ces poches sont généralement placées quelque part autour de l'appareil et représentent un risque de fuite lequel peut être contrôlé par la présence de ce bac de rétention.

En outre, l'invention propose une boîte comprenant un système de levage convenant pour réaliser un dispositif tel que précédemment décrit.

Le dispositif selon l'invention permet de protéger l'objet qu'il contient durant son stockage et son transport que ce soit sur des courtes distances (transport pédestre par exemple) ou sur de plus longues distances (transport en voiture, camion ou avion par exemple) grâce à sa boîte de protection.

Il permet également de faciliter la réception et la mise en service (mise en place, utilisation effective, rangement) de l'objet par son utilisateur. En effet, le système de levage du dispositif selon l'invention permet de lever l'objet sans effort pour le sortir de sa boîte et l'utiliser avant de le remettre sans plus d'efforts dans la boîte pour le ranger ou pour le transporter.

Ce dispositif facilite également les transports d'un point de vue pratique, en particulier dans les modes de réalisation où il est équipé d'au moins une poignée télescopique et de roulettes, pour un transport pédestre par exemple, et/ou d'au moins une poignée fixe pour faciliter le chargement dans un véhicule de transport par exemple.

Il peut de plus être conçu pour être entièrement réutilisable et ainsi éviter d'être source de déchets encombrants.

L'invention est avantageusement applicable à des objets fragiles qui nécessitent d'être régulièrement déplacés comme par exemple des appareils médicaux ou appareils de soins à domicile, notamment des appareils de dialyse.

L'invention concerne également des dispositifs comprenant toutes sortes d'objets, qui ne sont pas nécessairement en rapport avec le domaine médical.

L'invention concerne en particulier un dispositif comprenant tout objet relativement fragile, lourd et/ou volumineux, et dont l'utilisation est, de préférence, ponctuelle. On peut citer par exemple le cas de dispositifs de mesure en extérieur.

L'invention sera mieux comprise à la lecture de la description qui suit et des figures.

### BREVE DESCRIPTION DES FIGURES

La figure 1 est une représentation en perspective d'un dispositif selon un mode de réalisation de l'invention dans sa configuration ouverte et en position d'utilisation.
La figure 2 est une représentation en perspective du dispositif de la figure 1 dans sa configuration fermée, typiquement pour un transport de longue distance.
Les figures 3a et 3b sont des représentations en perspective du dispositif selon la figure 1 dans sa configuration fermée, typiquement pour un transport de courte distance.
La figure 4 est une représentation en perspective du dispositif selon la figure 1 dans sa configuration ouverte, en position de transport de courte distance (poignée télescopique sortie, boîte inclinée).
La figure 5 représente une forme d'exécution du couvercle selon l'invention.
Les figures 6a et 6b illustrent une forme d'exécution du système de levage du dispositif selon l'invention.

### DESCRIPTION DETAILLEE DE L'INVENTION

L'invention propose un dispositif 1 comprenant une boîte 2 et au moins un objet 4 destiné à être logé dans ladite boîte 2, ladite boîte 2 comprenant une ouverture 5 pour laisser passer ledit au moins un objet 4 et un système de levage 3, ledit système de levage 3 étant conçu pour recevoir ledit au moins un objet 4 de manière fixe et permettant i) de sortir ledit au moins un objet 4 de la boîte 2 en le faisant passer à travers ladite ouverture 5, typiquement pour l'utiliser, et ii) de rentrer ledit au moins un objet 4 entièrement dans la boîte 2, typiquement pour le ranger ou pour le transporter.

On entend par « boîte » 2 tout contenant comprenant une ouverture 5 et dans lequel peuvent être introduits un système de levage 3 ainsi qu'au moins un objet 4 fixé sur ledit système de levage.

On entend par objet 4, un objet, par exemple un appareil, composé d'un ou plusieurs éléments, le cas échéant fonctionnant de manière conjointe et complémentaire. Lesdits un ou plusieurs éléments étant conçus pour être transportés et utilisés en restant fixés au système de levage 3 de la boîte 2.

On entend par « système de levage » 3 tout moyen capable de recevoir au moins un objet 4 de manière fixe et permettant, alternativement, i) de sortir l'objet 4 de la boîte 2 en le faisant passer à travers ladite ouverture 5, et ii) de rentrer l'objet 4 entièrement dans la boîte 2. Le système de levage 3 sert également de support pour l'objet 4.

On entend par « de manière fixe » le fait que l'objet 4 reste fixé au système de levage 3 que le dispositif 1 selon l'invention soit en configuration ouverte ou en configuration fermée. L'objet 4 s'utilise donc en restant fixé au système de levage 3.

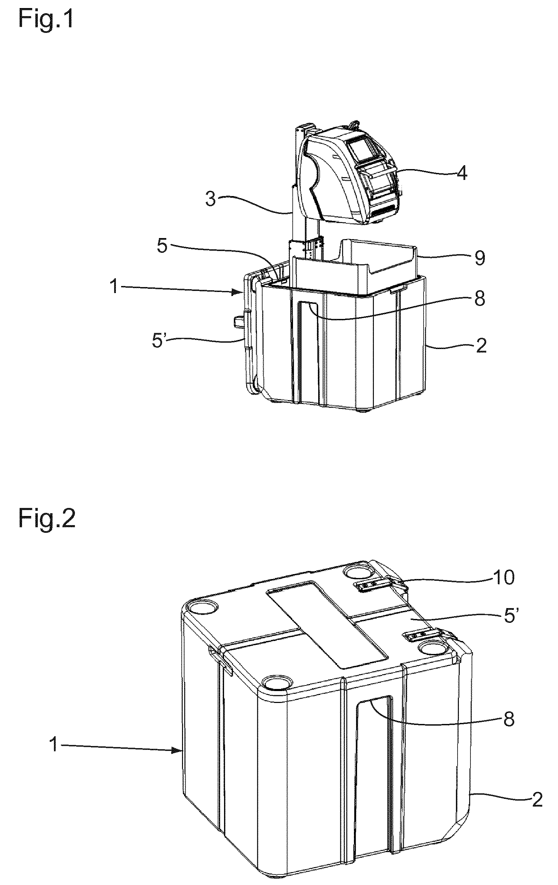

Selon un mode de réalisation préféré de l'invention, la boîte 2 comprend un couvercle 5' agencé pour fermer l'ouverture 5 de la boîte 2. De préférence, ce couvercle 5' est conçu avec des moyens de fermeture qui permettent de tenir le couvercle 5' en position fermée sur la boîte 2 lorsque le dispositif est en configuration dite « configuration fermée », comme illustré dans la figure 2. Il s'agit de la configuration dans laquelle se trouve le dispositif 1 pour le rangement ou pour les transports, en particulier pour les transports de longue distance.

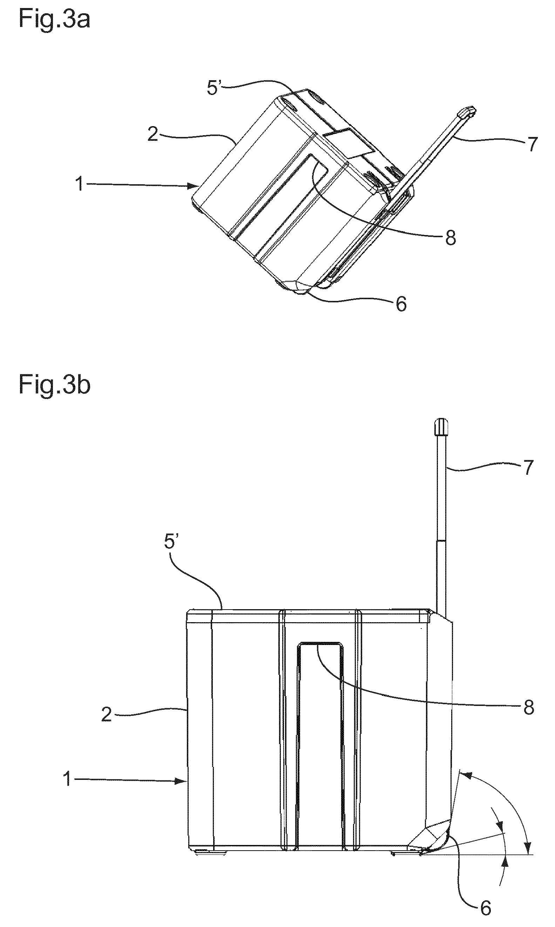

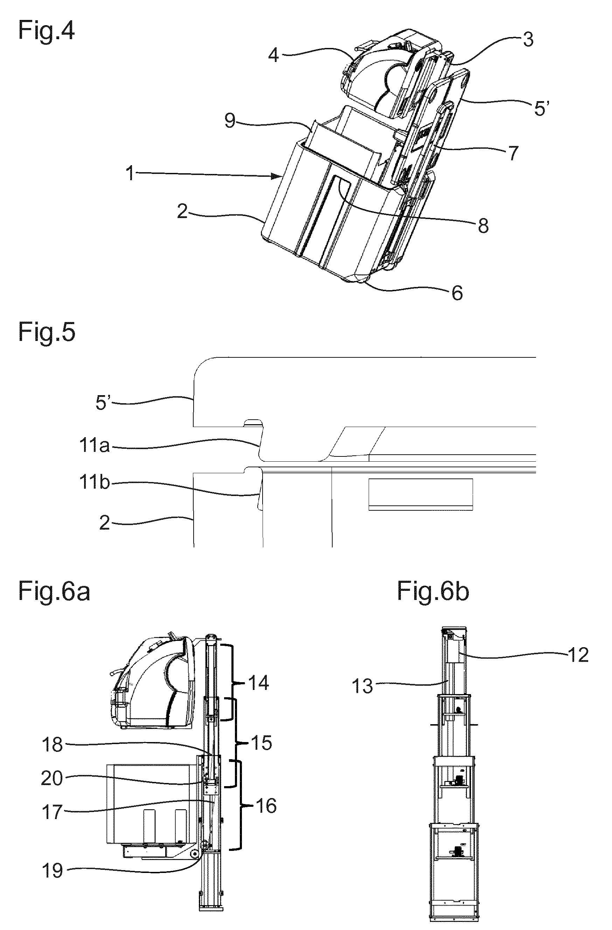

Cette configuration fermée est aussi utilisée pour les transports de courte distance, comme illustré dans les figures 3a et 3b, par exemple pour les déplacements au sein d'un logement. Cependant, un transport de courte distance est possible même lorsque le dispositif 1 est en configuration ouverte avec l'objet 4 en position d'utilisation, comme illustré dans la figure 4.

Lorsque le couvercle 5' est en position ouverte, le dispositif selon l'invention est dans une configuration dite « configuration ouverte ». Le dispositif 1 se trouve dans cette configuration dès lors que la boîte 2 n'est pas fermée par son couvercle 5', en particulier lors de son utilisation, comme illustré notamment dans les figures 1 et 4.

De préférence, le système de levage 3 est réalisé de manière à ce que la hauteur de l'objet 4 hors de la boîte 2 soit réglable et donc adaptable à l'utilisateur et à la position de celui-ci lors de l'utilisation de l'objet 4.

De préférence, la boîte 2 comporte au moins un moyen de commande du système de levage 3 qui permet de piloter la sortie de l'objet 4, typiquement pour l'utiliser, le réglage de la hauteur désirée de l'objet 4, typiquement pour l'utiliser, et la rentrée de l'objet 4 dans la boîte 2, typiquement pour le ranger ou pour le transporter.

Le système de levage 3 peut être réalisé d'une manière quelconque et employer n'importe quel type d'énergie, notamment manuelle ou via un moteur électrique 12. Le cas échéant, le moteur électrique 12 peut être alimenté soit par le biais d'une batterie soit directement sur le secteur.

Avantageusement la boîte 2 du dispositif 1 selon l'invention est, au moins en partie, moulée aux formes de l'objet 4 à protéger, ce qui permet, d'une part, d'assurer de bas coûts de fabrication et, d'autre part, de maintenir l'objet 4 en place dans la boîte 2.

Dans un mode de réalisation préféré de l'invention, la boîte 2 est réalisée dans une matière synthétique propre à absorber les chocs et les vibrations dues au transport, par exemple en polypropylène expansé (PPE). Des boîtes 2 réalisées dans d'autres matériaux, par exemple en aluminium couvert de mousse, sont évidemment possibles et l'invention n'est en aucun cas limitée à un mode de réalisation préféré.

Selon un mode de réalisation préféré de l'invention, le couvercle 5' de la boîte 2 est de la même matière que la boîte 2 elle-même et est maintenu fermé grâce à des moyens de fermeture, de préférence intégrés à la boîte 2 et au couvercle 5'. Lesdits moyens de fermeture sont typiquement constitués d'une forme 11a dont la contre-forme 11b existe dans la boîte 2, comme illustré dans la figure 5, afin d'éviter des moyens de fermeture supplémentaires.

Dans un mode de réalisation préféré de l'invention, le couvercle 5' est maintenu à la boîte 2 par des charnières 10 lors de son ouverture de manière à ne pas être perdu lors du transport ou de l'utilisation et ainsi garantir le transport suivant sans devoir approvisionner une autre boîte 2 ou une partie de celle-ci.

De plus, la fermeture du couvercle 5' assure de préférence une légère pression sur l'objet 4 qui est propre à garantir le maintien de celui-ci en position dans la boîte 2 y compris en cas de choc.

Au besoin, la boîte 2 fermée par son couvercle 5' peut être étanche à l'eau afin que l'objet 4 ne soit pas détérioré si, par exemple, le dispositif 1 était laissé à l'extérieur sous la pluie.

Dans un mode de réalisation préféré de l'invention, la boîte 2 du dispositif 1 comporte des roulettes 6. Avantageusement, lesdites roulettes 6 ne dépassent pas de la surface de la boîte 2 lorsque celle-ci repose sur l'une quelconque de ses faces, comme illustré dans la figure 2, mais permettent de faire rouler ladite boîte 2 lorsque celle-ci est inclinée, par exemple entre 14 et 80 degrés, comme illustré dans la figure 7.

Avantageusement, la boite 2 comporte également une poignée télescopique 7 permettant au dispositif 1 d'être incliné et tiré, notamment pour les déplacements de courtes distances au sein d'un logement par exemple. De tels déplacements sont possibles que la boîte 2 soit en configuration fermée, ou en configuration ouverte, et ceci même lorsque le dispositif 1 est en position d'utilisation.

Lorsqu'elles sont présentes, la poignée télescopique 7 et les roulettes 6 sont, de préférence, intégrées à la boîte 2 pour ne pas être arrachées lors des transports longue distance. Un enfoncement dans la boîte 2 permet de protéger la poignée télescopique 7 lorsque celle-ci est en position rentrée, c'est-à-dire lorsqu'elle n'est pas utilisée.

En outre, la boîte 2 du dispositif 1 selon l'invention comprend avantageusement au moins une, de préférence deux, poignées fixes 8.

Lesdites poignées fixes 8 sont avantageusement formées dans la boîte 2 elle-même et permettent de soulever le dispositif 1 par exemple pour le mettre dans une voiture. D'autres réalisations sont envisageables et le procédé de fabrication du dispositif 1 est adaptable en conséquence.

L'objet 4, et ses éventuelles parties supplémentaires (bac de rétention 9 par exemple), peut être de forme et de manufacture quelconque.

Sans limitation de ce qui précède, un objet 4 composé de plusieurs parties peut voir celles-ci levées simultanément ou non, complètement ou en partie.

Selon un mode de réalisation préféré de l'invention, représenté dans la figure 6, le système de levage utilise un moteur électrique 12 qui fait tourner une vis 13 qui entraine un premier élément mobile 14. Les deux autres éléments mobiles (15, 16) suivent le premier grâce à un système de câbles (17, 18) et poulies (19, 20). A l'évidence, l'invention ne se limite pas à ce mode préféré et des systèmes utilisant des vérins à air ou hydrauliques et un nombre quelconque d'éléments mobiles et/ou de moyens mécaniques font aussi partie de l'invention.

Le dispositif selon l'invention permet de protéger l'objet 4 durant le transport, de le lever sans effort pour le sortir de la boîte 2 et l'utiliser avant de le remettre sans plus d'efforts dans la boîte 2 pour le transport suivant.

Il est avantageusement réalisé de manière à permettre des transports de longue distance, par exemple en voiture, en camion ou en avion, grâce à sa boîte de protection réalisée pour absorber les chocs et les vibrations, mais aussi des transports de plus courtes distances, par exemple par transport pédestre, grâce à sa poignée télescopique 7 et à ses roulettes 6 intégrées à la boîte 2.

L'invention est en particulier avantageusement applicable à des appareils délicats qui nécessitent d'être régulièrement déplacés comme par exemple les appareils médicaux ou appareils de soins à domicile.

Un exemple d'application particulièrement avantageux est un dispositif de dialyse à domicile selon l'invention qui s'applique aux patients ayant une défaillance rénale chronique. La dialyse consiste à faire circuler, au moyen de pompes, le sang du patient dans un dialyseur pourvu d'une membrane semi-perméable. De l'autre côté de la membrane on circule un liquide appelé « dialysat » qui va attirer à lui les impuretés contenues dans le sang du fait de la différence de concentration présente de part et d'autre de la membrane selon un procédé connu appelé « diffusion ». Ce traitement nécessite un appareil qui gère les débits de sang et de dialysat ainsi que la sécurité du patient, un dialyseur, des poches qui contiennent du dialysat frais (typiquement entre 15 et 40 litres par traitement) et d'autres qui recueilleront le dialysat sorti du filtre. Ce traitement se déroule en général de 2 à 4 heures tous les jours ou tous les 2 jours selon la situation du patient. Dans certains cas il se fait de nuit, pendant environ 8 heures alors que le patient dort. Il en résulte que, le plus souvent, le patient n'est pas connecté à son appareil de dialyse, qui peut, grâce à la présente invention, être facilement rangé hors de sa vue. Cela permet au patient de faire abstraction de sa pathologie en dehors des heures de traitement ce qui allège le poids que celle-ci fait porter sur le patient lui-même et sur son éventuel entourage.

Dans cet exemple de réalisation particulier, le dispositif de dialyse selon l'invention comprend avantageusement un bac de rétention 9 sous l'appareil de dialyse dans lequel les poches de dialysat peuvent être déposées. Ce bac de rétention 9 limite les risques en cas de fuite et permet ainsi au patient une utilisation du dispositif dans les diverses pièces du logement. Avantageusement, ce bac de rétention 9 peut être placé sur des pesons, reliés à l'appareil, qui permettent de déterminer de manière précise les variations de poids des liquides écoulés et du patient lors du traitement.

Le bac de rétention 9 peut également, sans limitation de l'invention être réalisé par la boîte 2 elle-même.

De plus, dans un mode de réalisation préféré de l'invention, le système de levage 3 de ce dispositif permet d'ajuster la hauteur de l'appareil de dialyse ainsi que celle du bac de rétention 9 de sorte que la hauteur des poches de dialysat et celle de l'appareil de dialyse peuvent toutes deux être ajustées en fonction de la taille et de la position du patient.

## Revendications

1. Dispositif (1) comprenant une boîte (2) et au moins un objet (4) destiné à être logé dans ladite boîte (2), ladite boîte (2) comprenant une ouverture (5) pour laisser passer ledit au moins un objet (4) et un système de levage (3), ledit système de levage (3) étant conçu pour recevoir ledit au moins un objet (4) de manière fixe et permettant i) de sortir ledit au moins un objet (4) de la boîte (2) en le faisant passer à travers ladite ouverture (5), typiquement pour l'utiliser, et ii) de rentrer ledit au moins un objet (4) entièrement dans la boîte (2), typiquement pour le ranger ou pour le transporter.

2. Dispositif (1) selon la revendication 1, **caractérisé en ce que** la boîte (2) comprend en outre un couvercle (5') agencé pour fermer l'ouverture (5) de la boîte (2).

3. Dispositif (1) selon l'une quelconque des revendications précédentes, **caractérisé en ce que** le système de levage (3) permet de régler la hauteur de l'objet (4) hors de la boîte (2).

4. Dispositif (1) selon l'une quelconque des revendications précédentes, **caractérisé en ce que** la boîte (2) comporte au moins un moyen de commande permettant de piloter le système de levage (3).

5. Dispositif (1) selon l'une quelconque des revendications précédentes, **caractérisé en ce que** la boîte (2) est, au moins en partie, moulée aux formes dudit au moins un objet (4).

6. Dispositif (1) selon l'une quelconque des revendications précédentes, **caractérisé en ce que** la boîte (2) est fabriquée dans une matière synthétique propre à absorber les chocs et les vibrations dues au transport.

7. Dispositif (1) selon la revendication 6 **caractérisé en ce que** ladite matière synthétique propre à absorber les chocs et les vibrations dues au transport est du polypropylène expansé (PPE).

8. Dispositif (1) selon l'une quelconque des revendications précédentes, **caractérisé en ce que** la boîte (1) comporte des roulettes (6).

9. Dispositif selon la revendication 8, caractérisé en ce lesdites roulettes (6) ne dépassent pas de la surface de la boîte lorsque celle-ci repose sur l'une quelconque de ses faces.

10. Dispositif (1) selon l'une quelconque des revendications 8 et 9 **caractérisé en ce que** la boite (2) comporte également une poignée télescopique (7).

11. Dispositif (1) selon l'une quelconque des revendications précédentes, **caractérisé en ce que** la boîte (2) comporte au moins une poignée fixe (8).

12. Dispositif (1) selon l'une quelconque des revendications précédentes, **caractérisé en ce que** le système de levage (3) comporte un moteur électrique (12).

13. Dispositif (1) selon l'une quelconque des revendications précédentes, **caractérisé en ce que** ledit au moins un objet (4) est un appareil médical, par exemple un appareil de dialyse.

14. Dispositif (1) selon la revendication 13, **caractérisé en ce que** la boîte (2) comprend en outre un bac de rétention (9) porté par le système de levage (3) et situé sous ledit appareil médical.

15. Boîte (2) comprenant un système de levage (3) convenant pour réaliser un dispositif (1) selon l'une quelconque des revendications précédentes.
